# EUROPEAN PATENT APPLICATION

(11) **EP 3 995 811 A1**
(43) Date of publication of application: **11.05.2022**
(21) Application number: 20206697.3
(22) Date of filing: 10.11.2020
(51) Int. Cl.: G01N 21/11, G01J 3/44, G01N 21/64, G01N 21/65, G01N 33/18

(54) **CLEANING AND CALIBRATION OF AN ANALYSIS SYSTEM**

(71) Applicant: Artha AG, 8952 Schlieren (CH)
(72) Inventor: PFIFFNER, Peter, 8704 Herrliberg (CH); DENZLER, Daniel, 6030 Ebikon (CH)
(74) Representative: Zimmermann & Partner Patentanwälte mbB

(57) **Abstract**

Embodiments of an analysis system for analysing of components in a test liquid and a method for analysing of components in a test liquid are provided herein. The analysis system comprises a sample chamber adapted for holding the test liquid to be analysed; a liquid supply system for supplying the test liquid to the sample chamber and for supplying a cleaning liquid to the sample chamber; and a spectroscopy system for spectroscopic analysis of the test liquid being held in the sample chamber. Further, the analysis system comprises a controller adapted for controlling the analysis system to supply the cleaning liquid to the sample chamber; and perform, by the spectroscopy system, spectroscopic analysis of the cleaning liquid being held in the sample chamber thereby obtaining measured spectroscopic cleaning liquid data. The controller is further adapted to determine a deviation of the measured spectroscopic cleaning liquid data from an expected spectroscopic cleaning liquid data stored in the controller; and output, based on the determined deviation, a deviation indicator indicative of the determined deviation.

## Description

### FIELD OF THE INVENTION

The present disclosure relates to an analysis system for analysing of components in a test liquid. Further embodiments relate to a method for analysing of components in a test liquid.

### BACKGROUND OF THE INVENTION

Large-scale industrial applications involving the treatment or production of liquids or in another manner including liquids for manufacturing purposes usually require some form of quality control of the liquids, i.e. a continuous monitoring of the cleanliness and the type and concentrations of components or contaminants in the liquid. Examples of such large-scale industrial applications are sewage water treatment, and the food, beverage or drug industry. The quality control for such large-scale industrial applications is particularly demanding due to the high concentrations of components, or contaminants, being contained in the liquid samples. State-of-the-art methods for the quality control of test liquids include classical chemical analysis techniques.

Sewage water analysis is an application for which it is apparent that current state-of-the-art methods for qualitative and quantitative analysis of test samples are non-satisfactory. Sewage water treatment plants have to use elaborated cleaning stages in addition to mechanical and biological treatments to remove environmentally problematic contaminants.

Typical processes are based on initial treatment stages for removing larger contaminants (macro pollutants) as well as partially removing micro pollutants such as sand filtration, and are further based on micropollutant treatment stages such as membrane processes, osmosis, ozonolysis or active carbon filters. Sewage water treatment plants require monitoring and managing the performance of all cleaning stages (such as the exemplary aforementioned cleaning stages). Currently, the only available real-time measurement is UV-absorbance spectrometry, which has very limited chemical selectivity. Chemically selective information can currently only be obtained from ex-situ, lab-based measurements of (sewage) water samples. A fast feedback to react to spontaneously occurring pollutants is thus impossible.

Another major problem in state-of-the-art methods applied in large scale industrial production facilities is the need to monitor the cleanliness and maintain a certain level of cleanliness in the analysis system to obtain reliable results for qualitative and quantitative analysis of test samples. Many industrial applications, including the aforementioned fields, include high concentrations of components or contaminants in the process liquids, which result in depositions, the growth of biofilms and the like in the analysis system. Monitoring the cleanliness currently requires additional steps including the provision of reference substances and obtaining reference spectra for these reference substances to evaluate the cleanliness of the analysis system.

### BRIEF SUMMARY OF THE INVENTION

In view of the above, there is a need for a system and a method to perform qualitative and quantitative analysis of test liquids over extended periods of time, while, in particular automatically, monitoring a level of cleanliness of the analysis system in a simple and reliable manner, even for demanding industrial applications involving highly contaminated liquids.

Briefly, an analysis system and a method for analysing of components in a test liquid are provided to overcome at least some of the abovementioned limitations. This object is accomplished by means of an analysis system according to claim 1 and a method according to claim 15.

According to an embodiment of the present disclosure, an analysis system for analysing of components in a test liquid is provided. The analysis system comprises a sample chamber adapted for holding the test liquid to be analysed; a liquid supply system for supplying the test liquid to the sample chamber and for supplying a cleaning liquid to the sample chamber; and a spectroscopy system for spectroscopic analysis of the test liquid being held in the sample chamber. Further the analysis system comprises a controller adapted for controlling the analysis system to supply the cleaning liquid to the sample chamber; perform, by the spectroscopy system, spectroscopic analysis of the cleaning liquid being held in the sample chamber thereby obtaining measured spectroscopic cleaning liquid data. The controller is further adapted to determine a deviation of the measured spectroscopic cleaning liquid data from an expected spectroscopic cleaning liquid data stored in the controller; and output, based on the determined deviation, a deviation indicator indicative of the determined deviation.

According to another embodiment of the present disclosure, a method for analysing of components in a test liquid is provided. The method comprises an analysis system having a sample chamber adapted for holding the test liquid to be analysed; a liquid supply system for supplying the test liquid to the sample chamber and for supplying a cleaning liquid to the sample chamber; and a spectroscopy system for spectroscopic analysis of the test liquid being held in the sample chamber. The method comprises supplying the cleaning liquid to the sample chamber; and performing, by the spectroscopy system, spectroscopic analysis of the cleaning liquid being held in the sample chamber thereby obtaining measured spectroscopic cleaning liquid data. The method further comprises determining a deviation of the measured spectroscopic cleaning liquid data from an expected spectroscopic cleaning liquid data; and outputting, based on the determined deviation, a deviation indicator indicative of the determined deviation.

### BRIEF DESCRIPTION OF THE DRAWINGS

The parts shown in the Figures are not necessarily to scale, instead emphasis being placed upon illustrating the principles of the invention. Moreover, in the Figures, like reference signs designate corresponding parts. In the drawings:
Figure 1 schematically illustrates an analysis system according to an embodiment of the present disclosure.
Figure 2 schematically illustrates a spectroscopy system according to an embodiment of the present disclosure.
Figure 3 displays an example of a spectrum recorded with the second measurement set-up of the analysis system according to an embodiment of the present disclosure.
Figure 4 displays an example of a spectrum recorded with the first measurement set-up of the analysis system according to an embodiment of the present disclosure.

### DETAILED DESCRIPTION OF THE INVENTION

In the following detailed description, reference is made to the accompanying drawings, which form a part hereof, and in which are shown by way of illustration specific embodiments in which the invention may be practiced.

As used herein, the terms "having", "containing", "including", "comprising" and the like are open ended terms that indicate the presence of stated elements or features, but do not preclude additional elements or features.

It is to be understood that other embodiments may be utilised and structural or logical changes may be made without departing from the scope of the present invention. The following detailed description, therefore, is not to be taken in a limiting sense, and the scope of the present invention is defined by the appended claims. The embodiments described herein use specific language, which should not be construed as limiting the scope of the appended claims.

According to a general aspect of the present disclosure, the analysis system is an automatic or automated analysis system. The term "automatic" is to be understood such that - if appropriate after an initialisation step, which for example may comprise providing the test liquid in a sample chamber and/or starting a software application - the analysis of components (e.g., contaminants or other substances dissolved or suspended in the liquid) in the test liquid is conducted without the necessity for a user to engage with the system. This includes any steps of measurement and analysis with the spectroscopy system as well as calibration steps, but likewise any steps of supplying and removing the test liquid and the cleaning liquid to and from the sample chamber by means of the liquid supply system and any steps of cleaning or rinsing the sample chamber and/or the liquid supply system. The analysis system is provided in such a manner that, after installation, there is no need for a user to engage with any major parts of the system, apart from possibly adjusting software settings, such as for example determining freely selectable time intervals at which measurements should be carried out.

According to another general aspect of the present disclosure, the analysis system is preferably an inline analysis system. The term "inline" or "inline measurement" is to be understood such that the test liquid is provided in a flow-through system. The analysis system is configured to draw the test liquid from a large (industrial) scale liquid flow or large (industrial) scale liquid reservoir and release the test liquid after the analysis is concluded to an outlet. The analysis system is in fluid communication with the large (industrial) scale liquid flow or the large (industrial) scale liquid reservoir.

According to another general aspect of the present disclosure, the analysis system is preferably an on-site analysis system. The term "on-site" describes that the analysis of the test liquid is conducted in the close vicinity, for example less than 100 m or even less than 30 m, of the large (industrial) scale liquid flow or the large (industrial) scale liquid reservoir to be analysed. The analysis system is preferably not intended as an off-site laboratory for analysis of previously collected test liquids.

According to another general aspect of the present disclosure, the analysis system is a real-time analysis system. The term "real-time" describes that the measurement and the analysis of the test liquid are concluded rapidly, and the user is provided with information on the analysis results for the test liquid rapidly, for example within less than 10 minutes, and preferably less than 1 minute.

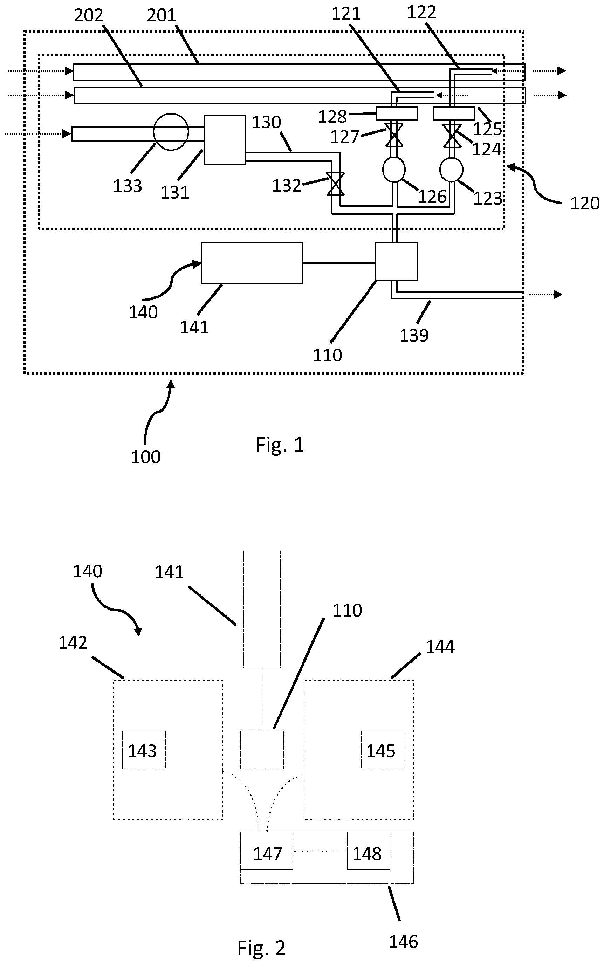

Figure 1 schematically illustrates part of an analysis system 100 for analysing of components in a test liquid, in particular the liquid supply system 120 and the sample chamber 110, according to an embodiment of the present disclosure. The analysis system 100 comprises a sample chamber 110, a liquid supply system 120, a spectroscopy system 140, and a controller (not displayed in Figure 1).

The liquid supply system 120 is adapted for supplying the test liquid to the sample chamber 110 and for supplying a cleaning liquid to the sample chamber 110. The liquid supply system 120 may be fluidly and/or mechanically connectable or connected to a main stream 201, 202 or the liquid reservoir to be tested. In the embodiment illustrated in Figure 1, the liquid supply system 120 is fluidly connected to a first main stream 201 and a second main stream 202. For example, the first main stream 201 may be a sewage water inflow stream (e.g. before carrying out a sewage water treatment step) and the second main stream 202 may be a sewage water outflow stream (e.g. after carrying out a sewage water treatment step). Further, the liquid supply system 120 may be fluidly and/or mechanically connectable or connected to a cleaning liquid supply stream or cleaning liquid supply reservoir (in Figure 1 the cleaning liquid supply is not shown, but indicated by the third dotted arrow from the top on the left-hand side of Figure 1). The liquid supply system 120 may further be fluidly and/or mechanically connectable or connected to an outlet stream or outlet reservoir for releasing the test liquid (in Figure 1 the cleaning outlet is not shown, but indicated by the third dotted arrow from the top on the right-hand side of Figure 1). The main stream and the outlet stream may also be identical in some embodiments.

The liquid supply system 120 typically is a liquid supply manifold. The liquid supply system 120 may include a plurality of tube or pipe sections suitable for liquids. Each of the pipe sections are preferably in fluid communication with another, unless fluid communication is blocked by a valve system. The liquid supply system 120 is in fluid communication with the sample chamber 110. Further, the liquid supply system 120 may include one or more test liquid supply channels. In Figure 1, the analysis system includes a first and a second test liquid supply channel.

The test liquid supply channel typically includes a branch off channel 121, 122, optionally (downstream of the branch off channel) a test liquid filter 125, 128, and (downstream of the branch off channel and the test liquid filter) a test liquid pump 123, 126. The branch off channel 121,122 may be arranged within the main stream 201, 202 for drawing the test liquid from the main stream 201, 202. The optional test liquid filter 125, 128 is configured for filtering out large scale particles, particularly in case large scale particles are not of interest or unsuitable for the analysis of the test liquid or may potentially damage (e.g. cotton buds) the analysis system. The filters are configured such as to not tamper the results of the analysis of the test liquid (i.e. by only removing large scale objects or particles). Typically, the average pore size of the filters is no more than 50 µm.

According to one aspect, the test liquid pump 123, 126 is configured for pumping the test liquid out of the main stream 201 and into the sample chamber 110. The valve system may include a test liquid valve 124, 127. The test liquid valve 124, 127 may be arranged downstream of the branch off channel and the test liquid filter. In some embodiments the test liquid valve 124, 127 is arranged downstream of the test liquid pump 123, 126, in others upstream.

Figure 1 displays a first test liquid supply channel with a first branch off channel 121 arranged within a first main stream 201, a first test liquid filter 128, a first test liquid valve 127 and a first test liquid pump 126. Further, Figure 1 illustrates a second test liquid supply channel with a second branch off channel 122 arranged within a second main stream 202, a second test liquid filter 125, a second test liquid valve 124 and a second test liquid pump 123.

The liquid supply system 120 typically includes a cleaning liquid supply branch 130. The cleaning liquid supply branch 130 typically includes an upstream opening, optionally a heater 131, and optionally a security valve 133, and a cleaning liquid pump (not shown in Figure 1). The upstream opening may be arranged within the cleaning liquid supply stream or cleaning liquid supply reservoir for drawing in the cleaning liquid.

According to an aspect, the heater 131 is arranged downstream of the upstream opening and is configured for heating the cleaning liquid before the cleaning liquid is supplied to the sample chamber 110. The heater 131 may be configured for heating the cleaning liquid to a temperature of at least 50 °C, preferably at least 60 °C, and more preferably at least 80 °C. The inventors observed that cleaning the analysis system with heated cleaning liquid facilitates the cleaning process substantially and reduces the necessity for using chemicals for cleaning the liquid supply system or the sample chamber.

According to an aspect, the cleaning liquid pump is configured for pumping the cleaning liquid out of the cleaning liquid supply stream or cleaning liquid supply reservoir and into the sample chamber 110. The valve system may include a cleaning liquid valve 132. The cleaning liquid valve 132 may be arranged downstream of the heater 131 and optionally downstream of the cleaning liquid pump.

The liquid supply system 120 may further include an outlet 139 in fluid communication with the sample chamber 110. The outlet 139 may be in fluid communication with the outlet stream or outlet reservoir for releasing the test liquid. Alternatively, the outlet may be also be in fluid communication with the main stream 201, 202 in some embodiments. The outlet 139 may also include an outlet valve (not shown in Figure 1).

According to an aspect, the valve system 124, 127, 132 is configured for opening and closing a test liquid flow of the test liquid to the sample chamber 110, for opening and closing a cleaning liquid flow of the cleaning liquid to the sample chamber 110; and optionally for opening and closing the outlet valve.

The inventors have identified further measures which enable maintaining the cleanliness of the sample chamber 110 and/or the liquid supply system 120 at an acceptable level over an extended period of time, thereby preventing the distortion of the spectroscopic analysis. As mentioned above, the liquid supply system 120 may comprise the heater 131 for heating the cleaning liquid before the cleaning liquid is supplied to the sample chamber 110. It was observed that cleaning the analysis system with heated cleaning liquid facilitates the cleaning process substantially and reduces the necessity for using chemical agents for cleaning the liquid supply system 120 or the sample chamber 110. In test runs it was observed that the quantity of contaminants in the sample chamber 110 could be reduced by approximately 30-50% by rinsing the sample chamber 110 once with heated cleaning liquid having a temperature of 50 - 80 °C. Typically, the cleaning liquid is only heated for some of the cleaning cycles, i.e. not every cleaning cycle is carried out with heated cleaning liquid.

Additionally, or alternatively, the liquid supply system 120 may include a pressuriser for pressurising the cleaning liquid, and a nozzle system adjacent to the sample chamber 110 for directing the pressurised cleaning liquid toward the sample chamber 110, in particular towards a transparent wall portion of the sample chamber 110.

The cleaning liquid is preferably chemically neutral and/or detergent-free. Chemically neutral is to be understood as approximately pH neutral. In a preferred embodiment, the cleaning liquid is free of chemical additives. Chemical additives are to be understood in this context as any substances added for the purpose of cleaning, disinfecting, or maintenance of the sample chamber 110.

Avoiding additives in the cleaning liquid enables limiting the costs of the cleaning cycles and inhibits additives from potentially precipitating or potentially forming precipitates together with contaminants in the sample chamber 110, which may not be easily removable from the sample chamber 110 and may negatively impact the accuracy of subsequent analysis cycles. Further, typical additives used for cleaning are detrimental to the environment and toxic. In a preferred embodiment, the cleaning liquid comprises water, and preferably essentially consists or even consists of water.

According to an embodiment, the analysis system 100 further includes an intransparent housing (not shown in Figure 1). The sample chamber 110 is fully arranged inside the intransparent housing. The liquid supply system 120 and/or the spectroscopy system 140 may also be arranged inside the intransparent housing. Preferably, the liquid supply system 120 and/or the spectroscopy system 140 are largely or even fully arranged within the intransparent housing. The intransparent housing is intransparent or configured to block radiation from the outside from impinging on the sample chamber 110 (and optionally on the liquid supply system 120 and/or the spectroscopy system 140) in the entire visible range, and preferably also the UV range having a wavelength of at least 200 nm, and/or the near infrared range having a wavelength of up to at least 1500 nm. Preferably, the intransparent housing is adapted for shielding against the entire wavelength range in which sunlight is significantly emitted. The inventors observed that the level of contaminants, in particular the quantity of biological contaminants, such as algae, may be significantly reduced by the provision of the intransparent housing. The intransparent housing therefore further facilitates in operating the analysis system 100 for an extended period of time without the need for servicing the analysis system 100. It was observed that algae growth could be largely or fully suppressed while operating the analysis system 100 for at least two weeks due to the provision of the intransparent housing. Even after four weeks of operation algae growth was modest.

The sample chamber 120 is adapted for holding the test liquid and the cleaning liquid, and is in fluid communication with the liquid supply system 120. The sample chamber 120 includes at least one transparent wall portion. The transparent wall portion is transparent for the wavelength range of an excitation beam of the spectroscopy system 140. Typically, the transparent wall portion is transparent for at least a wavelength range of 190 nm to 600 nm. The sample chamber 110 may include a second or further transparent wall portion for allowing the excitation beam to exit the sample chamber 110 and/or for allowing radiation emitted by the test liquid to reach a measurement set-up, and in particular a detector, of the spectroscopy system 140. The second or further transparent wall portion may be transparent for at least a wavelength range of 190 nm to 300 nm, or even at least a wavelength range of 190 nm to 600 nm. The second or further transparent wall portion may be arranged in a wall portion of the sample chamber 110 which is opposite to the transparent wall portion, or arranged in a side wall of sample chamber 110. The nozzle system for directing the pressurised cleaning liquid toward the sample chamber 110 may also be arranged in a side wall of the sample chamber 110. The cleanliness of the transparent wall portion and, if present, the second or further wall portions, can affect the accuracy of the spectroscopic analysis severely and may cause unreliable results in case the transparent wall portion contains deposits or biofilms, which may result in increased noise or interference signals in the spectroscopic analysis, such as scattered light or photoluminescence. The cleaning cycles may considerably reduce the amount of depositions and biofilms on the first or further transparent wall portions.

The spectroscopy system 140 is only indicated together with a radiation source 141 in Figure 1. Figure 2 schematically illustrates the spectroscopy system 140 in more detail according to an embodiment of the present disclosure.

The spectroscopy system 140 is provided for spectroscopic analysis of the test liquid and the cleaning liquid being held in the sample chamber 110. The spectroscopy system is an optical spectroscopy system. The spectroscopy system 140 includes a radiation source 141 for irradiating the test liquid in the sample chamber 110 by an excitation beam penetrating through the transparent wall portion. The radiation source 141 may emit radiation in the UV spectral range, in the visible range or in the near infrared spectral range. According to a preferred embodiment the radiation source 141 emits radiation in the UV range, and more preferably in the deep-UV range. The term "deep-UV" is to be understood as radiation with a wavelength between 190 nm and 270 nm. The excitation source 141 preferably emits radiation with a wavelength between 220 nm and 250 nm, and most preferably the wavelength is at least 230 nm and/or at most 250 nm. The radiation emitted by the excitation source 141 may be narrow in its wavelength range, i.e. the Full width at half maximum (FWHM) may be substantially less than 1 nm. The excitation source 141 may be a laser source, wherein the laser source is preferably selected from the group consisting of a microchip laser unit, a solid state laser, a gas laser, or a hollow cathode ion laser.

The spectroscopy system 140 includes a first measurement set-up 142 and/or a second measurement set-up 144, and optionally a third or further measurement set-ups.

In a preferred embodiment, as illustrated in Figure 2, the spectroscopy system 140 includes the first measurement set-up 142 for measuring photoluminescence radiation, and/or a second measurement set-up 144 for measuring a Raman spectrum.

According to an aspect, the second measurement set-up 144 is configured for measuring inelastically scattered radiation caused by the excitation source 141 and having been inelastically scattered by the test liquid. The second measurement set-up 144 may include a second detector 145. The second detector 145 may be any spectrometer, in particular the second detector 145 may be a Michelson spectrometer or a Fourier-transform spectrometer. By irradiating the test liquid with a wavelength in the deep-UV wavelength range, the intensity of Raman signals can be considerable due to the intensity of elastically scattered radiation being nonlinearly dependent on the radiation frequency or photon energy. Thus, Raman spectroscopy carried out in the deep-UV can result in signal intensities which are orders of magnitude higher than signal intensities observed in "classic" Raman measurements, which commonly employ excitation sources with wavelengths in the visible to near-infrared wavelength range. Advantageously, the wavelength of the radiation source 141 can be chosen such that resonant Raman spectroscopy can be realised, which may result in even more considerable signal intensities. Therefore, the second measurement set-up 144 based on deep-UV Raman spectroscopy can be highly sensitive and therefore especially suitable for detecting components or contaminants with very low concentrations.

One of the drawbacks of carrying out Raman measurements at wavelengths shorter than the commonly employed visible to near-infrared wavelength range may be the occurrence of fluorescence radiation, wherein fluorescence signal intensities can be substantially greater than Raman signal intensities. However, empirically it was found that fluorescence signal intensities are very moderate or even negligible for most components when radiation sources 141 with a wavelength in the deep-UV are employed, preferably with wavelengths no larger than 250 nm. Suitable radiation sources 141 with wavelengths in the deep-UV wavelength range and in particular with wavelengths below 250 nm, which generate sufficient photon flux have only become technically feasible recently.

According to an aspect, the first measurement set-up 142 is suitable for measuring photoluminescence radiation emitted by the test liquid as a result of absorption of radiation by the test liquid, and in particular for detecting radiation with a wavelength between 260 nm and 600 nm. The first measurement set-up 142 may include a first detector 143. The first detector 143 may be any spectrometer, in particular the first detector 143 may be a Michelson spectrometer or a Fourier-transform spectrometer, or an optical grating.

In some embodiments, the spectroscopy system 140 may further include a third measurement set-up. The third measurement set-up may be configured for measuring a further optical property, in particular an opacity of a transparent wall portion of the sample chamber 110.

According to an embodiment of the present disclosure, the spectroscopy system 140 may further comprise a signal processing unit 146. As a result of conducting a measurement in the second measurement set-up 144 a data array R(λ,l) is generated. As a result of conducting a measurement in the first measurement set-up 142 a data array A(λ,l) is generated. The signal processing unit 146 comprises a pre-processing unit 147 configured for receiving data generated by the first measurement set-up 142 and by the second measurement set-up 144. The pre-processing unit 147 may receive data due to an interconnection between each of the first and second measurement set-ups 142, 144 and the pre-processing unit 147 and may be based on a WiFi connection, a Bluetooth connection, a physical wire or on a printed circuit board. Furthermore, in embodiments comprising a third measurement set-up or possibly even further measurement set-ups, the pre-processing unit 147 may also be configured for receiving data generated by the third measurement set-up and/or further measurement set-ups.

The signal processing unit 146 may further include a machine learning algorithm 148 for determining, from the data generated by the first measurement set-up 142 and by the second measurement set-up 144, qualitative and quantitative information regarding the composition of the test liquid or the cleaning liquid. The data may be analysed by the machine learning algorithm 148 employing a Convolutional Neural Network (CNN) or an artificial neural network (ANN) or principle component analysis (PCA) or any other statistical method suitable for recognising features and/or patterns. The machine learning algorithm 148 may be configured for background noise reduction and/or noise subtraction. The background noise reduction and/or noise subtraction may be carried out automatically and/or implicitly by the machine learning algorithm 148, preferably by the Convolutional Neural Network, based on a layer for feature recognition. This is contrary to typical analysis methods, which are based on modelling background noise by means of a mathematical function and subtracting this mathematical function from the data prior to processing the data further. The signal processing unit 146 according to embodiments of the present disclosure may be configured for simultaneous background noise reduction and/or noise subtraction and one or more further data processing steps.

The machine learning algorithm 148 may carry out an automatic feature recognition, wherein the machine learning algorithm 148 can be trained on raw, uncorrected data, i.e. without carrying out a background noise reduction and/or noise subtraction. According to an embodiment, the machine learning algorithm 148 may employ a Convolutional Neural Network (CNN), wherein the Convolutional Neural Network (CNN) comprises the following layers: a deep network with input layer, a plurality of convolution layers for automatic feature recognition and a plurality of fully connected layers for classification with a final output layer. Furthermore, in embodiments comprising a third measurement set-up or possibly further measurement set-ups, the data generated by the third measurement set-up and/or possible further measurement set-ups may be used as additional input for the machine learning algorithm 148, which advantageously can help to increase a prediction precision of the automatic feature recognition. The machine learning algorithm can be improved and/or extended and/or replaced with a different algorithm or model, for example if new data becomes available or new anomalies are detected, on a regular base by a software update. Software updates may be automatic updates of the Convolutional Neural Network facilitated by a data update comprising loading a new configuration and/or definition, for example based on the open neural network exchange (ONNX) format or a custom-made format. A simple data update may advantageously circumvent the need for installing new software. Further details of features of the machine learning algorithm 148 may be found in Liu et al. (Analyst, 2017, 142, 4067-4074) and/or Zou et al. (Scientific Reports 7, 2017, Article number: 6186), which are both herein incorporated by reference in their entirety.

In samples, wherein multiple components contribute to a measurement, a plurality of signals may overlap and thus encumber the interpretation of the measurement. Advantageously, by employing the machine learning algorithm 148 the feature recognition may result in reliable qualitative and quantitative information for each individual component in the test liquid.

The analysis system 100 according to embodiments of the present disclosure may be chemically selective and yield quantitative results regarding the concentration of components in the test liquid even when a plurality of different components are present in the test sample, without the need for separation of individual components of the test sample prior to carrying out measurements with the analysis system 100, which may reduce the amount of time and effort required to analyse the test liquid. Advantageously, the analysis system 100 may substantially reduce the time required to carry out an analysis of the test sample and can be configured for on-site measurement of the test sample. Furthermore, due to short measurement times for the first and second measurement set-ups 142, 144 and rapid analysis of the data by the signal processing unit 146, the analysis of the test sample may be carried out, and in particular displayed, in real-time.

Having described the sample chamber 110, the liquid supply system 120 and the spectroscopy system 140, including preferred embodiments and preferred aspects, the following description provides details on the controller or control system. The controller is adapted for controlling the analysis system 100. The controller is adapted to control the liquid supply system 120, including supplying the test liquid and/or the cleaning liquid to the sample chamber 110. In particular, the controller is adapted for controlling each of the pumps of the liquid supply system 120 and the valve system 124, 127, 132. Further, the controller is adapted to control the spectroscopy system 140, including performing, by the spectroscopy system 140, spectroscopic analysis of the test liquid (during analysis cycles) and/or the cleaning liquid (during cleaning cycles) being held in the sample chamber 110.

For example, the controlling to carry out the analysis cycle may comprise: controlling the valve system 124, 127, 132 to open the test liquid flow for supplying the test liquid to the sample chamber 110; controlling the spectroscopy system 140; and controlling the valve system 124, 127, 132 to close the test liquid flow for stopping the flow of the test liquid to the sample chamber 110. The step of closing the test liquid flow may be carried out before, during or after the step of controlling the spectroscopy system. In other words, the controller may be adapted to provide the test liquid as a continuous flow, i.e. with the spectroscopy system being configured for carrying out a spectroscopic analysis of the test liquid while the test liquid is flowing through the sample chamber 110 or alternatively the controller may be adapted to supply the test liquid to the sample chamber, subsequently to close off the test liquid flow and then to control the spectroscopy system 140 to carry out a spectroscopic analysis of the test liquid carry. The controlling to carry out the cleaning cycle may include the same steps as the controlling to carry out the analysis cycle.

The present inventors discovered that the cleaning liquid utilised during cleaning cycles can additionally be used for diagnosing a level of cleanliness of the sample chamber 110 or in other words diagnose whether the cleanliness is sufficient to carry out a further analysis cycle. The analysis system according to embodiments of the present disclosure may also be referred to as self-calibrating (or self-monitoring) analysis system. In addition, the cleaning liquid may be utilised to diagnose whether the analysis system, and in particular the spectroscopy system 140 is fully functional. Prior art analysis systems and methods utilise reference substances, which are introduced in the sample chamber in order to assess whether the cleanliness is sufficient or the spectroscopy system is fully functional. Examples of such reference substances for UV applications are acetonitrile, gallium nitride or Teflon. Prior art analysis systems require draining the cleaning liquid, providing a separate reference substance supply and introducing the reference substance to the sample chamber, performing one or more test measurements with the reference substance, removing the test substance and subsequently performing additional cleaning cycles to remove possible residuals of the reference substance in the sample chamber. The present invention substantially simplifies the prior art techniques by requiring less components, requiring less time to diagnose a level of cleanliness, requiring less servicing (due to potential issues with the reference substance or supplying the reference substance), and reducing the costs. The present invention is particularly advantageous for an automated analysis system, in which both cleaning cycles and diagnosing a level of cleanliness are carried automatically. The present invention is also particularly advantageous for applications including highly contaminated test liquids (or test liquids with a high concentration of components), such as sewage water. The demanding applications that the present disclosure relates to can already result in a significant contamination of the sample chamber 110 and/or the liquid supply system 120 after carrying out very few analysis cycles. Contamination of the sample chamber 110 impairs and potentially also distorts the results of the spectroscopic analysis. Specifically, contamination of the sample chamber may be substantial after just one analysis cycle, and thus cleaning cycles are typically required after every analysis cycle (every measurement of the test liquid), and a level of cleanliness may need to be determined after every analysis cycle.

The controller is adapted to perform, by the spectroscopy system 140, spectroscopic analysis of the cleaning liquid being held in the sample chamber 110 thereby obtaining measured spectroscopic cleaning liquid data. The spectroscopic analysis may be performed with just one measurement set-up of the spectroscopy system, or alternatively with more than one measurement set-up. According to one embodiment, spectroscopic analysis of the cleaning liquid is performed with the first measurement set-up 142 for measuring photoluminescence radiation.

Although the first, second or third measurement set-ups may be adapted or designed for measuring a specific spectroscopical property, in particular a specific optical spectroscopic property, the (first, second or third) measurement set-up may further be suitable for detecting other types of spectroscopical signals for which the measurement set-up is not primarily optimised for. For example, a measurement set-up for measuring photoluminescence radiation may be primarily suitable for detecting a photoluminescence signal, however in the recorded spectrum other contributions, e.g. from Raman signals or elastically scattered radiation may also be visible. Illustratively, the first measurement set-up 142 for measuring photoluminescence radiation may be suitable for detecting radiation with a wavelength between 260 nm and 600 nm. The measured spectrum ranging from 260 nm to 600 nm may include further signals, e.g. Raman signals, besides the photoluminescence signals. Likewise, a measurement set-up 144 for measuring a Raman spectrum may be primarily suitable for detecting a Raman signal, however in the recorded spectrum other contributions, e.g. from fluorescence signals may also be visible. In other words, the (first, second or third) measurement set-ups may be adapted to detect a first type of signal (for which the measurement set-up is primarily adapted) and a second type of signal. The spectroscopy system 140 may be configured such that the first type of signal and the second type of signal are detected by the respective measurement set-up in separate, and preferably non-overlapping, wavelength ranges. Illustratively, Raman signals may also be detected by the first measurement set-up in a wavelength range of approximately 260 to 270 nm, whereas photoluminescence signals may be detected at a wavelength of larger than 270 nm.

The spectroscopic cleaning liquid data may be obtained or derived from the first measurement set-up 142 for measuring photoluminescence radiation. The spectroscopic cleaning liquid data may correspond to the full measured spectrum recorded with the first measurement set-up 142 or a portion of the measured spectrum. According to one exemplary embodiment, the spectroscopic cleaning liquid data is derived from the full spectral range recorded with the first measurement set-up, e.g. 260 nm to 600 nm. The spectroscopic cleaning liquid data may then correspond to a two-dimensional array data array A(λ,l) including the wavelength and the intensity of the measured spectrum.

In another exemplary embodiment, the measured spectroscopic cleaning liquid data comprises, or preferably essentially consists of, a measured peak intensity of a predetermined spectroscopic peak of the cleaning liquid. According to this exemplary embodiment, the spectroscopic cleaning liquid data may be obtained by predetermining the wavelength range to take into account the predetermined spectroscopic peak, and integrating the intensities detected in the predetermined wavelength range. In this exemplary embodiment, the spectroscopic cleaning liquid data may therefore correspond to a scalar (integrated) intensity value. Alternatively, the predetermined wavelength range may extend beyond the predetermined spectroscopic peak, such as an additional 100 nm towards higher wavelength ranges, and the spectroscopic cleaning liquid data may correspond to a two-dimensional array data array A(λ,l).

According to one embodiment, the (first, second or third) measurement set-up is adapted to detect a first type of signal (for which the measurement set-up is primarily adapted) and a second type of signal, wherein the predetermined spectroscopic peak of the cleaning liquid corresponds to the second type of signal. Illustratively, the predetermined spectroscopic peak of the cleaning liquid may be a Raman peak. According to a preferred embodiment, the cleaning liquid comprises water, and more preferably essentially consists or consists of water. The predetermined spectroscopic peak may correspond to a Raman peak of water.

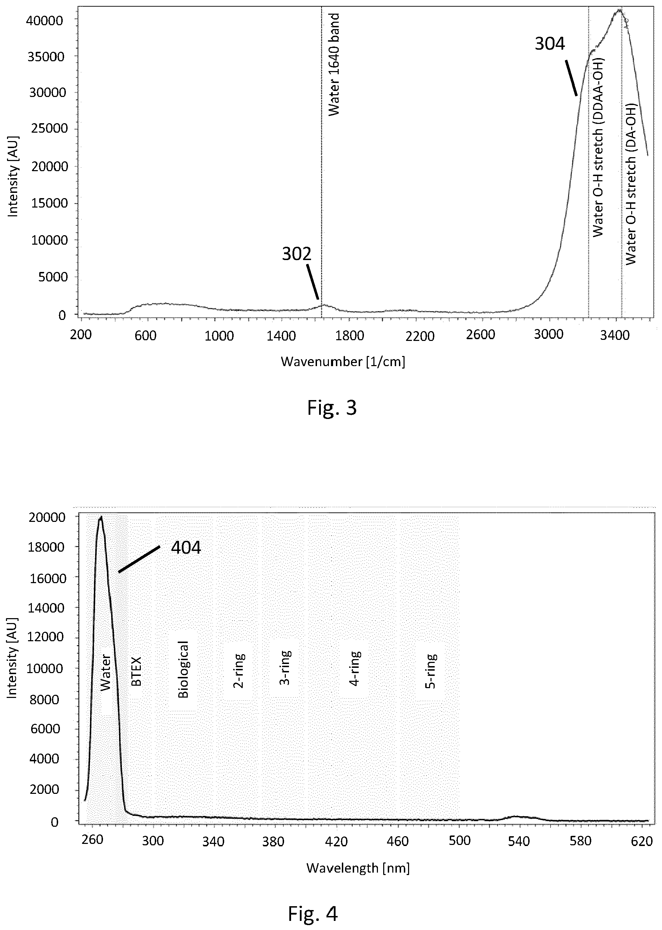

Figure 3 displays a spectrum of the cleaning liquid recorded with the second measurement set-up 144 for measuring a Raman spectrum. The cleaning liquid is water. The measured spectrum includes two peaks 302, 304. Peak 302 is visible at around 1640 cm⁻¹. Peak 304 extends from approximately 3000 cm⁻¹, peaks at approximately 3400 cm⁻¹, and includes two contributions each of which involve O-H stretching bands.

Figure 4 displays a spectrum of the cleaning liquid recorded with the first measurement set-up 142 for measuring photoluminescence radiation. One intense peak 404 is visible between approximately 260-280 nm. This peak originates from a Raman signal, i.e. corresponds to peak 304 shown in Figure 3. Water as cleaning liquid shows very minor intensity fluorescence signals (i.e. the intensities of the first type of signal is minor), whereas the Raman signal at approximately 3400 cm⁻¹ (or at approximately 270 nm for the wavelength of the radiation source selected in this specific example) is intense. Figure 4 additionally sketches wavelength ranges in which contaminants, such as BTEX (benzene, toluene, and the three xylene isomers), biological components, or 2-5 ring aromatic compounds, typically are observed in fluorescence spectra (especially when excited with a UV or deep UV radiation source). In the example shown in the Figure, the spectroscopic cleaning liquid data may be obtained or derived from the first measurement set-up 142 by utilising the full spectral range recorded, i.e. 260 nm to 600 nm, and the spectroscopic cleaning liquid data may correspond to a two-dimensional array data array A(λ,l) including the wavelength and the intensity of the measured spectrum. Alternatively, the measured spectroscopic cleaning liquid data may be obtained by incorporating the Raman peak only (and integrating over e.g. 260 to 280 nm); or by utilizing the spectral range around and including the Raman peak, and the spectroscopic cleaning liquid data may correspond to a two-dimensional array A(λ,l). The inventors discovered that in case of contamination of the sample chamber 110, and in particular contamination of the transparent wall portion of the sample chamber 110, the contributions of the first type of signal (fluorescence signal) may significantly increase (especially at wavelengths of 280 nm and higher), and the intensity of the second type of signal (Raman) signal may significantly decrease. Therefore, both the first and the second type of signal taken alone, as well as the combination of both the first and the second type of signals, yield information on the cleanliness of the sample chamber, and in particular of the transparent wall portion.

In an alternative embodiment, the spectroscopic cleaning liquid data may be obtained or derived from the second measurement set-up 144 for measuring a Raman spectrum. The spectroscopic cleaning liquid data may be obtained in a similar manner to that described above for the first measurement set-up. Typically, the Raman spectrum may only contain one type of signal with significant intensity. In this embodiment, the spectroscopic cleaning liquid data may typically essentially consist of one predetermined spectroscopic peak of the cleaning liquid, such as a Raman peak of water (see e.g. peak 304 in Figure 3). Although in this case, the contaminants in the sample chamber may not result in additional signals in the Raman spectrum (unless the sample chamber or the cleaning liquid is highly contaminated), it is observed that the Raman signals of the cleaning liquid may be significantly reduced in intensity already in the event of small or intermediate concentrations of components in the sample chamber, such as depositions on the transparent wall portion.

According to one embodiment, the spectroscopic cleaning liquid data is scaled by means of a spectroscopy system characterizing parameter. The analysis system 100 may include a sensor for measuring the spectroscopy system characterizing parameter. Scaling with the spectroscopy system characterizing parameter can help to ensure that any deviation observed in the measured spectrum of the cleaning liquid is caused by components in the sample chamber 110 and not by the spectroscopy system. One example may be a change in the output power of the radiation source. According to a preferred embodiment, the spectroscopy system characterising parameter is a radiation source characterising parameter. According to a preferred aspect, the radiation source characterising parameter is a power of the radiation emitted by the radiation source 141. For example, the intensities of the measured spectrum may be divided by the power of the radiation, measured with a power meter, to obtain the spectroscopic cleaning liquid data.

The controller is adapted to compare the measured spectroscopic cleaning liquid data with expected spectroscopic cleaning liquid data. The expected spectroscopic cleaning liquid data corresponds to a previous measurement carried out with the analysis system 100, including the same cleaning liquid, and preferably similar or identical measurement parameters used for the spectroscopy system 140. The previous measurement may be stored in the controller and processed in the same manner as the measured spectroscopic cleaning liquid data. In particular, the expected spectroscopic cleaning liquid data corresponds to the same spectral feature (such as the Raman peak disclosed above) or the same spectral range as used for the measured cleaning liquid data. Further, the expected spectroscopic cleaning liquid data may be obtained by scaling the measured data with the spectroscopy system characterising parameter, such as the power of the radiation source used for this specific measurement.

The controller is adapted to determine a deviation between the measured spectroscopic cleaning liquid data and the expected spectroscopic cleaning liquid data. The controller is adapted to output, based on the determined deviation, a deviation indicator indicative of the determined deviation. The deviation indicator may for example be a percentage deviation. In case the (measured/expected) spectroscopic cleaning liquid data is derived from a single spectral feature (such as the Raman peak disclosed above), the deviation indicator may be a single scalar value. In case an entire spectral range is used, the deviation indicator may correspond to an array of individual percentage deviations for each respective wavelength of that spectral range. According to a preferred aspect, the deviation is derived from the measured peak intensity, in particular the scaled peak intensity, and the expected peak intensity of the predetermined spectroscopic peak of the cleaning liquid data.

According to an embodiment, the controller is configured to compare the deviation indicator with a predetermined reliability condition. The predetermined reliability condition is a threshold, wherein if the deviation indicator equals or exceeds the reliability condition, the level of cleanliness is insufficient to perform spectroscopic analysis of the test liquid or at least the results of spectroscopic analysis of the test liquid will likely be unreliable. For example, the reliability condition may be a percentage deviation of at most 15%, preferably at most 10% and more preferably at most 5% (between measured spectroscopic cleaning liquid data and expected spectroscopic cleaning liquid data). In case the deviation is an array (by selecting a spectral range for evaluating the deviation), the predetermined reliability condition may be defined such that the reliability condition is exceeded as soon as one of the data points exceeds the aforementioned percentage deviation (e.g. the deviation between measured and expected data exceeds 15% for one specific wavelength value). Alternatively, the predetermined reliability condition may be defined such that the reliability condition is exceeded if the average of the sign of the deviations is at least 15%, preferably at least 10% and more preferably at least 5%. According to another aspect, the reliability condition can also include several types of conditions. For example, the reliability condition may include the aforementioned percentage deviation for spectroscopic cleaning liquid data and a percentage deviation for opacity data. The controller may be adapted to determine a deviation of the measured opacity cleaning liquid data from an expected opacity cleaning liquid data stored in the controller.

According to an aspect, the controller is adapted to issue a warning if the deviation indicator is not below the predetermined reliability condition. The controller may be adapted for repeatedly performing spectroscopic analysis of the cleaning liquid during a cleaning cycle. According to an aspect, the controller is adapted to issue a warning if the deviation indicator is not below the predetermined reliability condition after a predetermined cleaning time or after performing spectroscopic analysis of the cleaning liquid for a predetermined number of cycles.

According to an aspect, the controller is adapted for carrying out the cleaning cycle and repeatedly performing spectroscopic analysis of the cleaning liquid until the deviation indicator is below the predetermined reliability condition. This aspect is advantageously combined with the aspect related to issuing the warning. The controller may continuously issue the warning until the deviation indicator is below the predetermined reliability condition. The presence or absence of the warning indicates to a user that a subsequent analysis cycle of the test liquid may or may not be carried out or that the analysis system may require servicing.

Additionally, or alternatively the controller may be adapted to output the deviation indicator in a continuous manner during a cleaning cycle of the sample chamber 110. In a "continuous manner" is to be understood that the output is updated each time a more recent deviation indicator is determined. The controller may be adapted for determining a cleaning progress indicator derived from a plurality of consecutively determined deviation indicators. The output and/or the cleaning progress indicator may indicate to a user the progress of the cleaning cycle or in case no progress is achieved that servicing of the analysis system be required.

The controller may be configured for controlling the cleaning cycle in dependence on the deviation indicator. For example, in case the deviation indicator is substantial, and in particular exceeds the reliability condition, the controller may be configured to carry out one or more of the aforementioned steps to increase the level of cleanliness. For example, the controller may be adapted to increase the duration of the cleaning cycle and/or carry out an additional cleaning cycle before carrying out a subsequent analysis cycle.

According to an embodiment, the analysis system 100 is a waste water analysis system 100 and the test liquid is waste water. The analysis system 100 is particularly suitable for maintaining a high level of cleanliness even in the very demanding field of waste or sewage water and can be automatically operated long term without the need to service or replace components of the analysis system, such as the sample chamber 110 or the liquid supply system 120. Furthermore, the spectroscopic analysis can be carried out at any purification stage, thereby enabling monitoring the concentration of contaminants such as micropollutants, phosphor compositions or nitride. The analysis system 100 may be suitable for the analysis of water in water pipes, such as tap water pipes, sanitary pipes or heating pipes in houses or in the ground or water pipes for process heating, process cooling as well as commercial cooling systems and building cooling systems.

According to an embodiment, the analysis system 100 is a beverage and food water analysis system 100 and the test liquid is beverage water and/or food water. Similarly, as discussed above for the waste water industry, the analysis system 100 may be beneficial for analysing industrial production of food and/or beverages and/or drugs.

## Claims

1. An analysis system (100) for analysing of components in a test liquid, comprising:
a sample chamber (110) adapted for holding the test liquid to be analysed;
a liquid supply system (120) for supplying the test liquid to the sample chamber (110) and for supplying a cleaning liquid to the sample chamber (110);
a spectroscopy system (140) for spectroscopic analysis of the test liquid being held in the sample chamber (110); and
a controller adapted for controlling the analysis system (100) to
- supply the cleaning liquid to the sample chamber (110);
- perform, by the spectroscopy system (140), spectroscopic analysis of the cleaning liquid being held in the sample chamber (110) thereby obtaining measured spectroscopic cleaning liquid data;
- determine a deviation of the measured spectroscopic cleaning liquid data from an expected spectroscopic cleaning liquid data stored in the controller; and
- output, based on the determined deviation, a deviation indicator indicative of the determined deviation.

2. The analysis system according to claim 1, wherein the spectroscopy system (140) comprises a radiation source (141), wherein the radiation emitted by the radiation source (141) has a wavelength between 190 nm and 270 nm, preferably between 220 nm and 250 nm, and more preferably between 230 nm and 250 nm; and optionally wherein the radiation source (141) is a laser source.

3. The analysis system according to any one of the preceding claims, wherein the spectroscopy system (140) comprises
a first measurement set-up (142) for measuring photoluminescence radiation, in particular wherein the first measurement (142) set-up is suitable for detecting radiation with a wavelength between 260 nm and 600 nm; and/or
a second measurement set-up (144) for measuring a Raman spectrum.

4. The analysis system according to any one of the preceding claims, wherein the spectroscopic cleaning liquid data is derived from the first measurement set-up (142) for measuring photoluminescence radiation.

5. The analysis system according to any one of the preceding claims, wherein the spectroscopic cleaning liquid data is scaled by means of a spectroscopy system characterising parameter, preferably a radiation source characterising parameter, and more preferably a power of the radiation emitted by the radiation source (141).

6. The analysis system according to any one of the preceding claims, wherein the measured spectroscopic cleaning liquid data comprises, in particular essentially consists of, a measured peak intensity of a predetermined spectroscopic peak of the cleaning liquid.

7. The analysis system according to claim 6, wherein the predetermined spectroscopic peak of the cleaning liquid is a Raman peak.

8. The analysis system according to any one of claims 6 or 7, wherein the deviation is derived from the measured peak intensity, in particular the scaled peak intensity, and the expected peak intensity of the predetermined spectroscopic peak of the cleaning liquid data.

9. The analysis system according to any one of the preceding claims, wherein the controller is adapted for repeatedly performing spectroscopic analysis of the cleaning liquid during a cleaning cycle; and in particular wherein the controller is adapted to output the deviation indicator in a continuous manner during a cleaning cycle of the sample chamber (110).

10. The analysis system according to any one of the preceding claims, wherein the controller is adapted for carrying out the cleaning cycle until the deviation indicator is below a predetermined reliability condition; and/or wherein the controller is configured to issue a warning if the deviation indicator is not below the predetermined reliability condition after a predetermined cleaning time.

11. The analysis system according to any one of the preceding claims, wherein the controller is adapted for determining a cleaning progress indicator derived from a plurality of consecutively determined deviation indicators.

12. The analysis system according to any one of the preceding claims, wherein the cleaning liquid comprises water, preferably essentially consists or consists of water, and more preferably essentially consists or consists of distilled water.

13. The analysis system according to any one of the preceding claims, wherein the spectroscopy system (140) comprises a third measurement set-up for measuring a further optical property, in particular an opacity of a transparent wall portion of the sample chamber (110).

14. The analysis system according to any one of the preceding claims,
wherein the analysis system (100) is a waste water analysis system and wherein the test liquid is waste water; or
wherein the analysis system (100) is a beverage and/or food water analysis system and wherein the test liquid is beverage water and/or food water.

15. A method for analysing of components in a test liquid, comprising an analysis system (100) having
a sample chamber (110) adapted for holding the test liquid to be analysed;
a liquid supply system (120) for supplying the test liquid to the sample chamber (110) and for supplying a cleaning liquid to the sample chamber (110);
a spectroscopy system (140) for spectroscopic analysis of the test liquid being held in the sample chamber (110); and
the method comprising:
- supplying the cleaning liquid to the sample chamber (110);
- performing, by the spectroscopy system (140), spectroscopic analysis of the cleaning liquid being held in the sample chamber (110) thereby obtaining measured spectroscopic cleaning liquid data;
- determining a deviation of the measured spectroscopic cleaning liquid data from an expected spectroscopic cleaning liquid data; and
- outputting, based on the determined deviation, a deviation indicator indicative of the determined deviation.
